# EUROPEAN PATENT SPECIFICATION

(11) **EP 2 178 445 B1**
(45) Date of publication and mention of the grant of the patent: **03.10.2012**
(21) Application number: 08781840.7
(22) Date of filing: 15.07.2008
(51) Int. Cl.: A61B 17/04, A61B 17/08, A61B 17/10, A61B 17/11

(54) **RIVET INTRODUCTION SYSTEM**
NIETENEINFÜHRUNGSSYSTEM
SYSTÈME D'INTRODUCTION DE RIVET

(30) Priority: 17.07.2007 US 950209 P
(43) Date of publication of application: 28.04.2010
(73) Proprietor: Cook Medical Technologies LLC, Bloomington, IN 47404 (US)
(72) Inventor: SAEED, Zahid, A., Cincinnati, OH 45243 (US); SURTI, Vihar, C., Winston-Salem, NC 27104 (US); KENNEDY, Kenneth C., Clemmons North Carolina 27012 (US)
(74) Representative: Garratt, Peter Douglas
(86) International application number: PCT/US2008/070046
(87) International publication number: WO 2009/012250

(56) References cited:
- US-A1- 2001 039 436
- US-A1- 2005 228 413
- US-A1- 2006 264 986

## Description

### FIELD OF THE INVENTION

The present invention relates to a medical device for use in endoscopic surgery, and more particularly, to a device and system configured to introduce rivets for use in endoscopic or laparoscopic tissue repair.

### BACKGROUND

Tissue repair during endoscopic and laparoscopic procedures presents several difficulties. Given the size and mobility constraints associated with these minimally invasive surgical methods, there are special challenges to traditional tissue repair methods such as suturing. This problem has prompted the development of a number of endoscopic therapeutic approaches including the use of sutures, staples, clips, and similar devices. Although such approaches are often effective, each requires use of specialized equipment and techniques requiring accessory tools and/or particular dexterity. Open surgery to repair tissue injuries such as perforations or tears to gastric or intestinal tissues require invasive techniques that are associated with a higher morbidity rate and many other undesirable side effects. Although there are some functional treatment methods as mentioned, there exists a need for more effective procedures using minimally invasive surgical techniques.
US2006/0264986 discloses a medical device configured for introducing a blind rivet (in the meaning of the present application, see second paragraph of the detailed description below). The two part form of appended claim 1 is based on this disclosure.

### BRIEF SUMMARY

The present invention is a rivet introduction means as set out in appended claim 1.

### BRIEF DESCRIPTION OF THE DRAWINGS

FIG. 1A is a perspective view of an unexpanded first rivet embodiment;

FIG. 1B depicts a perspective view of the rivet embodiment of FIG. 1A in a single-expanded state;

FIG. 1C shows a perspective view of the rivet embodiment of FIG. 1A in a dual-expanded state;

FIG. 2A is an exterior view of a rivet introduction device embodiment;

FIG. 2B illustrates a longitudinal section view of the rivet introduction device of FIG. 2A;

FIGS. 3A-3F illustrate a method of using the rivet introduction device;

FIGS. 4A-4C show another rivet embodiment; and

FIGS. 5A-5D show still another rivet embodiment.

### DETAILED DESCRIPTION

The present invention includes a rivet introduction device which may be configured for use with an endoscope such as, for example, a duodenoscope. Those of skill in the art will appreciate that embodiments of the present invention may also be used with other minimally invasive surgical devices and systems such as, for example, a laparoscopy system including a laparoscope and one or more accessory trocars. Embodiments of the present invention may present advantages of ease of use and mechanical simplicity as compared to endoscopic suturing devices and other presently-existing devices configured for repairing or otherwise connecting tissue.

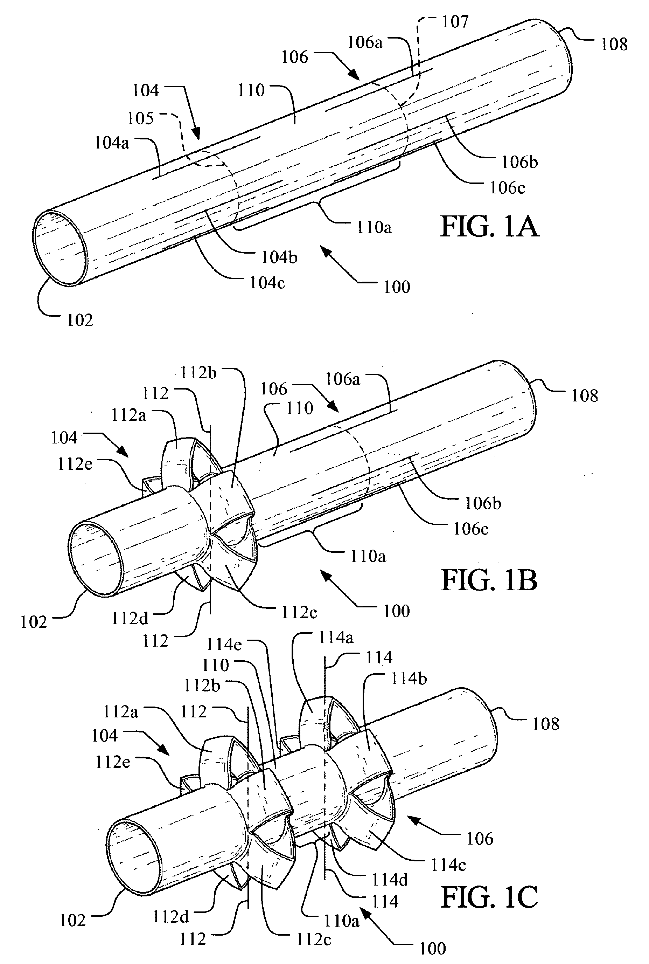

In embodiments of the present invention different rivet embodiments may be used with a rivet introduction device in a rivet introduction system. FIGS. 1A-1C show one embodiment of a rivet 100. The rivet 100 is configured as a blind rivet, by which it is meant that a user does not need direct access to a distal rivet end 102 to deploy first and second expansion zones 104, 106 of the rivet 100. In unexpanded form, the rivet 100 may be a generally cylindrical column with a central longitudinal rivet lumen (although those of skill in the art will appreciate that other geometries may be used within the scope of the present invention). As shown in FIG. 1A, the rivet 100 includes an elongate body portion 110 having a proximal rivet end 108 and a distal rivet end 102. The rivet 100 may be constructed of any material having appropriate rigidity and malleability qualities including, for example, a polymer, nitinol, or another alloy such as, for example, 304 stainless steel; further, the rivet may be constructed of any combination thereof such as, for example, a composite of plastic and metal (e.g., metal core coated by plastic, plastic capture zone with metal expansion regions or vice versa). The rivet 100 may also include visualization markers such as radio-opaque, luminescent, or other markings that will allow a user to visualize the rivet 100. The elongate body portion 110 includes the first expansion zone 104 and the second expansion zone 106, with a tissue capture zone 110a therebetween. Each of the expansion zones 104, 106 includes a series of apertures such as, for example, a series of slits 104a-n, 106a-n through the rivet wall (where "n" represents a letter corresponding to a highest-numbered aperture). In the illustrated embodiment, the series of slits 104a-n, 106a-n are generally parallel, but parallel structure is not required. As shown in FIG. 1A, each expansion zone 104, 106 may include a central region 105, 107 that is pre-stressed or otherwise configured to deform more readily than adjacent regions of the respective expansion zone.

FIG. 1B depicts a single-expanded configuration of the rivet 100, wherein the first expansion zone 104 has been expanded to form a first securement plane 112, which is disposed generally transverse to the longitudinal axis of the elongate body portion 110 along a line 112--112. The first securement plane 112 includes a series of petals 112a-n that have been folded outward between the series of slits 104a-n by a longitudinal compression of the elongate body portion 110 of the rivet 100. FIG. 1C depicts a double-expanded configuration of the rivet 100, wherein in addition to the first expansion zone 104 having been expanded, the second expansion zone 106 has also been expanded to form a second securement plane 114, which is disposed generally transverse to the longitudinal axis of the elongate body portion 110 along a line 114--114. The second securement plane 114 includes a series of petals 114a-n that have been folded outward between the series of slits 106a-n by a longitudinal compression of the elongate body portion 110 of the rivet 100. In some embodiments, the regions between the series of slits 104a-n, 106a-n may be pre-stressed such that they more readily deform to form the series of petals 112a-n, 114a-n.

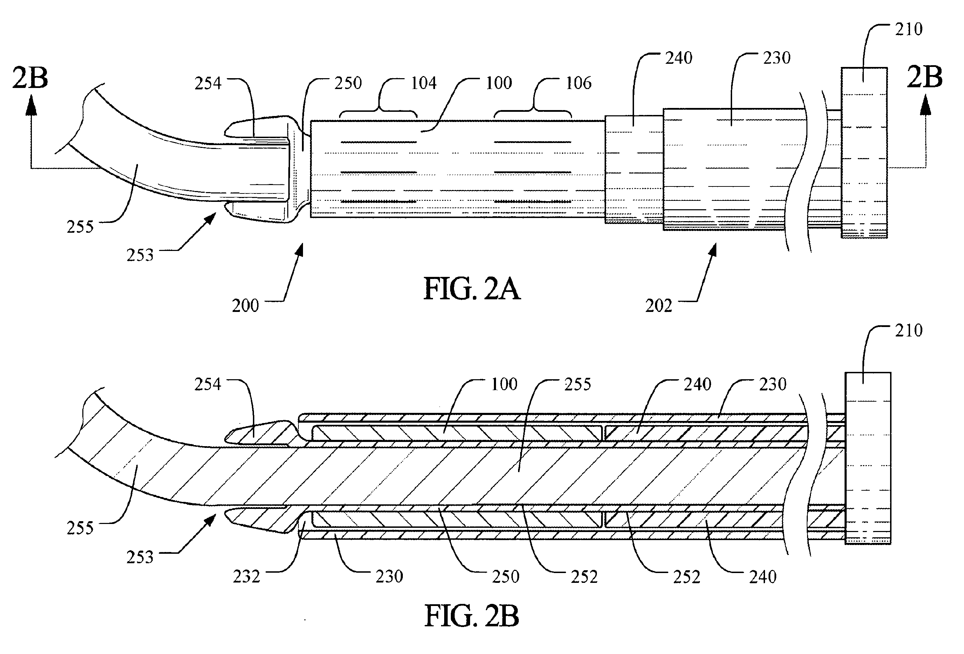

FIG. 2A illustrates a rivet introduction system 200 of the present invention, including a rivet introduction device 202 and a rivet 100 (of the type shown in FIGS. 1A-1C), with the components extended for visibility. FIG. 2B shows a longitudinal cross-section of the components in a non-extended configuration, such as might be used when introducing the rivet introduction system 200 through the working channel of an endoscope or through a trocar of a laparoscopy system. The rivet introduction device 202 includes a proximal handle 210 (shown only diagrammatically; those of skill in the art will appreciate that a standard or modified three-ring handle or other handle configuration currently known or developed in the future may be used within the scope of the present invention). An outer sheath 230 extends distally from the proximal handle 210 and is coaxially disposed around an inner sheath 240, which is longitudinally slidable through an outer sheath lumen 232. In the illustrated embodiment, the inner sheath 240 has about the same outer diameter as the rivet 100. The inner sheath 240 includes an inner sheath lumen 242 through which a mandrel 250 extends longitudinally. The mandrel 250 includes a guide wire lumen 252, through which a guide wire 255 is disposed.

A distal portion of the mandrel 250 includes a flexible mouth portion 253 having jaw members 254. As is also shown in FIG. 2A, when the guide wire 255 is extended between the jaw members 254, the jaw members 254 are spread apart such that the outer diameter of the flexible mouth portion 253 is greater than the inner diameter of the rivet 100. This configuration allows the mandrel 250 to hold the rivet 100 longitudinally against the inner sheath 240. There may be as few as two jaw members 254, but those of skill in the art will appreciate that a mandrel 250 may be configured to include three or more jaw members 254 that can open/close radially around a guide wire 255/ guide wire lumen mouth.

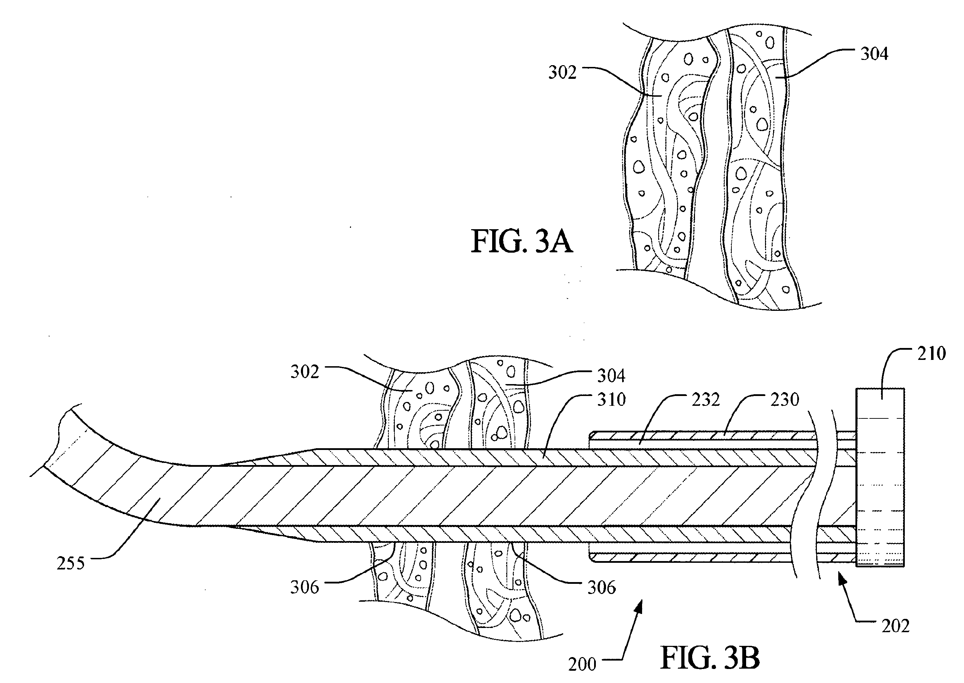

FIGS. 3A-3F illustrate a method of introducing a rivet 100 using the rivet introduction device 202 with reference to components shown in FIGS. 1A-2B. FIG. 3A shows first and second soft tissue structures 302, 304 to be connected. Those of skill in the art will appreciate that the presently described method will be applicable to a variety of tissue types and tissue-affixation settings such as, for example, repairing a tissue tear or lesion (e.g., transmural gastric lesion) or creating an anastomosis. Those of skill in the art will also appreciate that this method can be used in circumstances where only one side of tissues to be joined is accessible. As shown in FIG. 3B, a penetrating member 310 such as, for example, a "hot FNA needle" (a fine needle aspiration needle transmitting an electrical current for cutting and/or coagulation) or another appropriate needle may be directed through the first and second soft tissue structures 302, 304 to create an aperture 306 with the guide wire 255 introduced therethrough. The penetrating member 310 may be withdrawn, and the rivet introduction system 200 may be directed along the guide wire 255 to the tissue site to be treated.

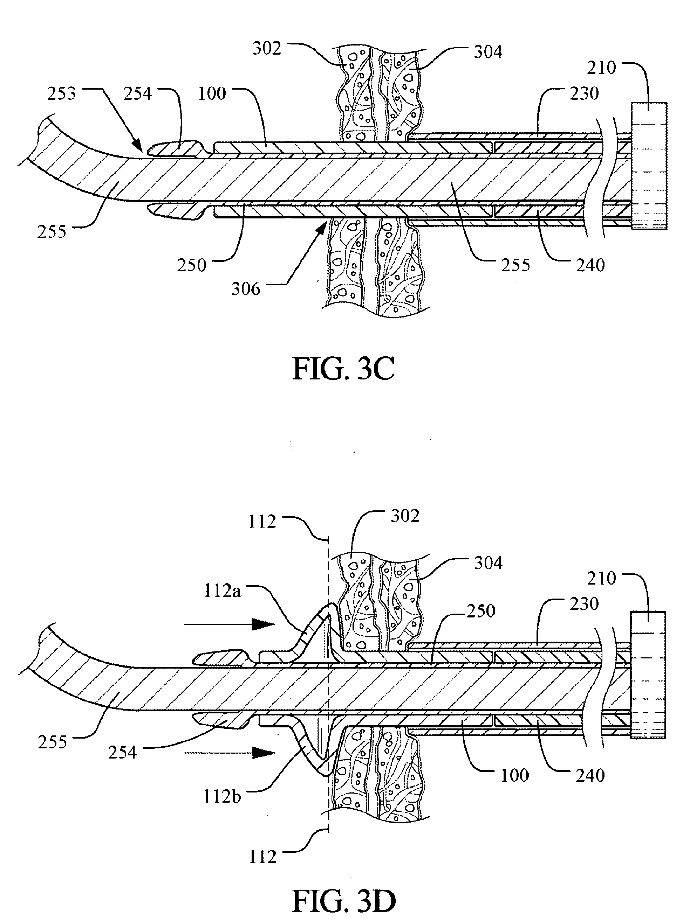

Then, as shown in FIG. 3C, the mandrel 250, together with the rivet 100 around the mandrel 250, may be directed through the aperture 306 such that the first expansion zone 104 (see FIG. 2A) is distal of the aperture 306. Next, as shown in FIG. 3D, the jaw members 254 of the mandrel 250 may be drawn proximally against the distal rivet end 102 with sufficient force to expand the series of petals 112a-n of the first expansion zone 104 to form the first securement plane 112 on the distal side of the first soft tissue structure 302. The distal end of the inner sheath 240 preferably prevents proximal movement of the rivet 100 such that the proximally-directed force of the mandrel 250 against the rivet 100 will expand the first expansion zone 104 rather than moving the rivet 100 proximally. The outer sheath 230 remains disposed around the second expansion zone 106 (see FIG. 2A), preventing it from expanding. The guide wire 255 is disposed through the flexible mouth portion 253 of the mandrel 250, keeping the flexible mouth portion 253 expanded so that the jaw members 254 contact the distal end of the rivet 100 with sufficient force to expand the first expansion zone 104. At this point, the entire assembly may be pulled proximally to position the first and second soft tissue structures 302, 304 in close contact.

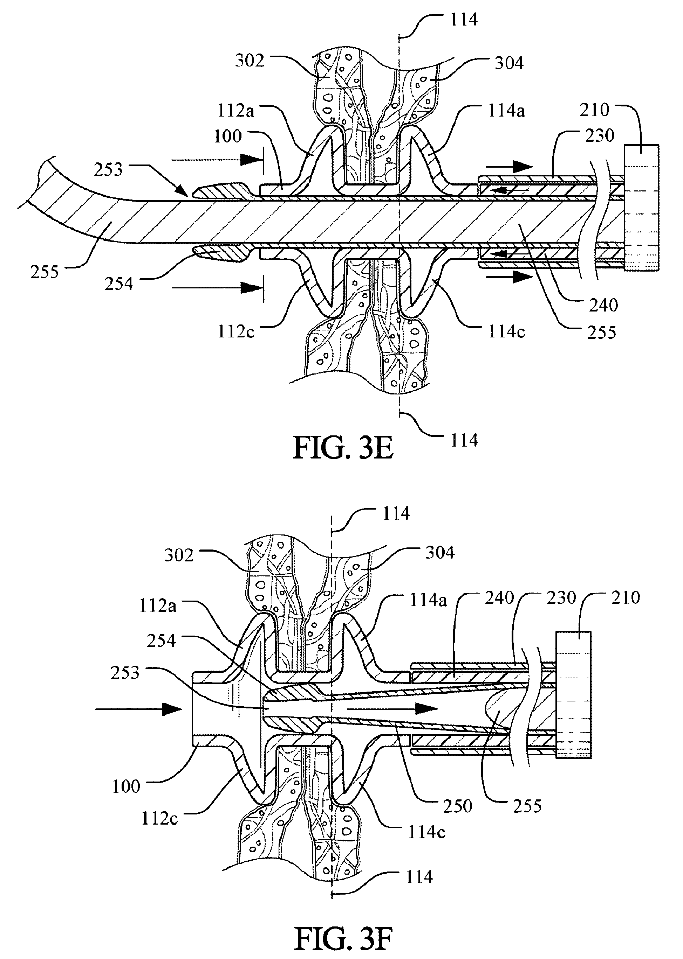

Next, as shown in FIG. 3E, the outer sheath 230 may be drawn proximally such that the second expansion zone 106 is exposed. Then, the mandrel 250 may be held stationary against the distal rivet end 102, while the inner sheath 240 is advanced distally with sufficient force to expand the series of petals 114a-n of the second expansion zone 106 to form the second securement plane 114 on the proximal side of the second soft tissue structure 304. Alternatively, the mandrel 250 may be drawn proximally, while the distal end of the inner sheath 240 preferably prevents proximal movement of the rivet 100 such that the proximally-directed force of the mandrel 250 against the rivet 100 will expand the second expansion zone 106 rather than move the rivet 100 proximally. In either of these manners, the first and second securement planes 112, 114 may secure the first and second soft tissue structures 302, 304 together.

After the first and second soft tissue structures 302, 304 are secured, the rivet introduction device 202 may be withdrawn. FIG. 3F shows that the guide wire 255 may be withdrawn to a location proximal of the flexible mouth portion 253 of the mandrel 250. Then, the mandrel 250 may be withdrawn through the rivet 100 as - without the guide wire 255 present to keep the flexible mouth portion 253 open - the outer diameter of the flexible mouth portion 253 can be collapsed to fit through the inner diameter of the rivet 100. As a final step, the rivet introduction device 202 may be withdrawn away from the rivet 100. Those of skill in the art will appreciate that visualization of the steps of this method may be accomplished by direct visualization using a camera or other viewing instrumentality of an endoscope, ultrasound, fluoroscopy, and/or a combination thereof. The size of the rivet 100 and its relative dimensions (e.g. lengths of tissue capture zone and expansion zones) may be selected based upon the dimensions and type(s) of the tissue(s) to be secured.

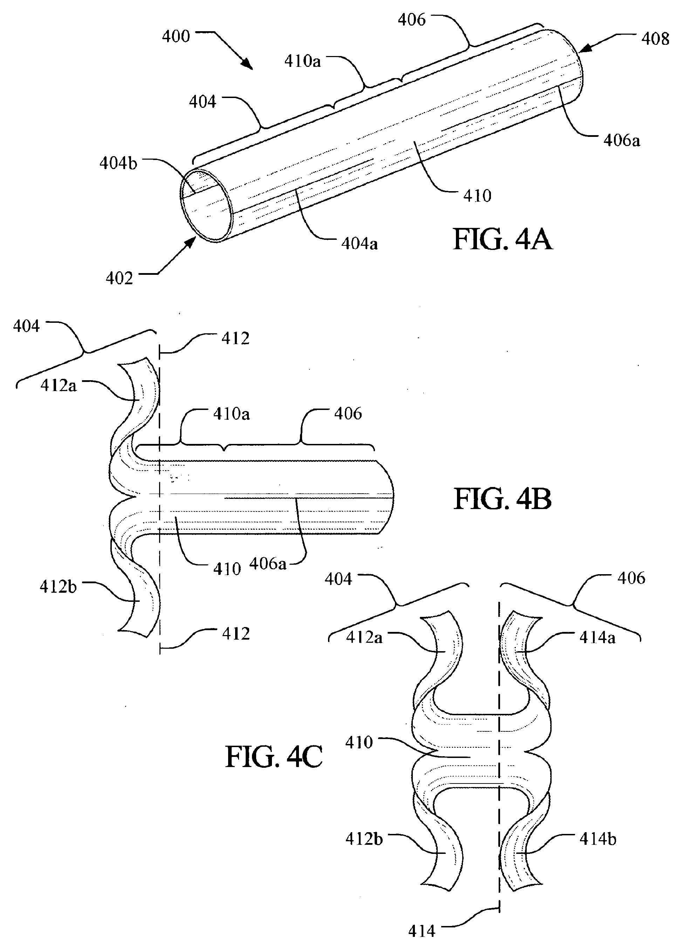

FIGS. 4A-4C show another embodiment of a rivet 400. The rivet 400 is configured as a blind rivet, by which it is meant that a user does not need direct access to a distal rivet end 402 to deploy first and second expansion zones 404, 406 of the rivet 400. In unexpanded form, the rivet 400 may be embodied as a generally cylindrical column with a central longitudinal rivet lumen (although those of skill in the art will appreciate that other geometries may be used within the scope of the present invention). As shown in FIG. 4A, the rivet 400 includes an elongate body portion 410 having a proximal rivet end 408 and a distal rivet end 402. The rivet 400 may be constructed of any material having appropriate rigidity and malleability qualities including, for example, a polymer, stainless steel, or another alloy. The rivet 400 may also include visualization markers such as radio-opaque, luminescent, or other markings that will allow a user to visualize the rivet 400. The elongate body portion 410 includes the first expansion zone 404 and the second expansion zone 406, with a tissue capture zone 410a therebetween. Each of the expansion zones 404, 406 includes a series of apertures such as, for example, a series of slits 404a-n 406a-n through the rivet wall (where "n" represents a letter corresponding to a highest-numbered aperture). In the illustrated embodiment, the series of slits 404a-n, 406a-n are generally parallel, but parallel structure is not required.

FIG. 4B depicts a single-expanded configuration of the rivet 400, wherein the first expansion zone 404 has been expanded to form a first securement plane 412, which is disposed generally transverse to the longitudinal axis of the elongate body portion 410 along a line 412--412. The first securement plane 412 includes a series of petals 412a-n that have been folded outward between the series of slits 404a-n by a longitudinal compression of the elongate body portion 410 of the rivet 400 (where, for 412a-n, "n" represents a letter corresponding to a highest-numbered petal). FIG. 4C depicts a double-expanded configuration of the rivet 400, wherein in addition to the first expansion zone 404 having been expanded, the second expansion zone 406 has also been expanded to form a second securement plane 414, which is disposed generally transverse to the longitudinal axis of the elongate body portion 410 along a line 414--414. The second securement plane 414 includes series of petals 414a-n that have been folded outward between the series of slits 406a-n by a longitudinal compression of the elongate body portion 410 of the rivet 400. In some embodiments, the regions between the series of slits 404an, 406a-n may be pre-stressed such that they more readily deform to form the series of petals 412a-n, 414a-n.

In one embodiment, the rivet 400 may be introduced and deployed in the same manner as illustrated with reference to FIGS. 3A-3F, using a mandrel 250 to expand the petals 412a-n, 414a-n. In another embodiment, the series of petals 412a-n, 414a-n may be constructed of a memory material such as a polymer and/or alloy that is biased into an expanded configuration. In a method for deploying a memory material embodiment of a rivet 400 using a rivet introduction device 202, the outer sheath 230 is kept around the first expansion zone 404 until it is in a position to be expanded (e.g., adjacent a distal surface of tissue being secured). Then the outer sheath 230 may be withdrawn, allowing the series of petals 412a-n of the distal first expansion zone 404 to open. Similarly, the second expansion zone 406 can be allowed to open by further retracting the outer sheath 230 when the second expansion zone 406 is in a desired position (e.g., adjacent a distal surface of tissue being secured), such that the series of petals 414a-n of the second expansion zone 406 are allowed to open.

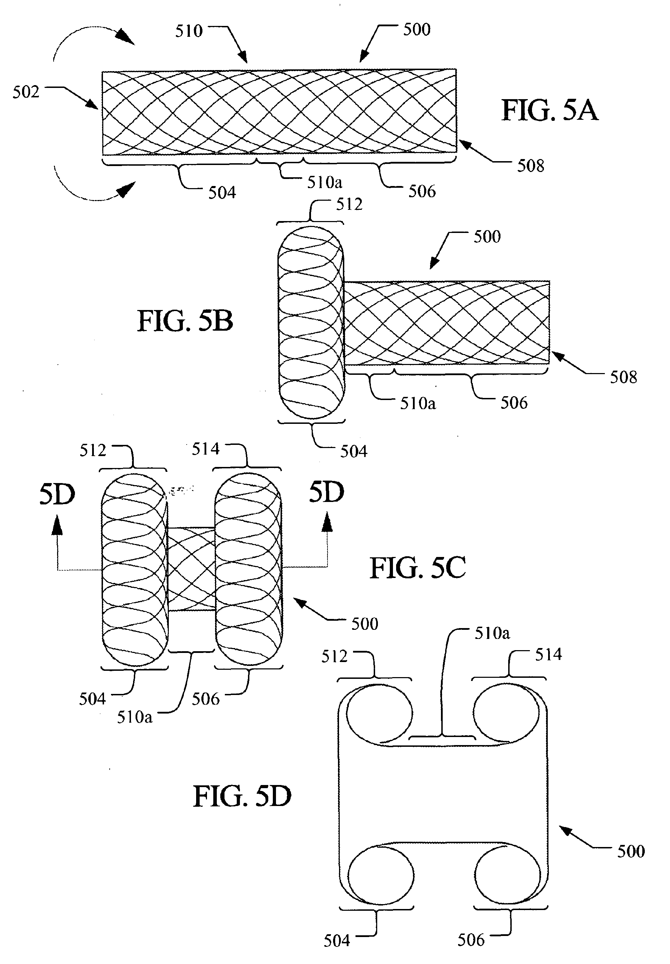

FIGS. 5A-5C show yet another embodiment of a rivet 500. The rivet 500 is configured as a blind rivet, by which it is meant that a user does not need direct access to a distal rivet end 502 to deploy first and second expansion zones 504, 506 of the rivet 500. In unexpanded form, the rivet 500 may be embodied as a generally cylindrical column constructed of a mesh or other wire arrangement similar to an open-sided stent with a central longitudinal rivet lumen (although those of skill in the art will appreciate that other geometries may be used within the scope of the present invention). In one embodiment, the rivet 500 may be constructed of a memory material or another material biased into the shape described below and shown in FIGS. 5A-5C, such that a deployment thereof does not require a mandrel. Additionally, the rivet 500 may be deployed in the manner described above (with reference to a memory material embodiment of the rivet 400).

As shown in FIG. 5A, the rivet 500 includes an elongate body portion 510 having a proximal rivet end 508 and a distal rivet end 502. The rivet 500 may be constructed of any material having appropriate rigidity and malleability qualities including, for example, a polymer, nitinol, or another alloy. The rivet 500 may also include visualization markers such as radio-opaque, luminescent, or other markings that will allow a user to visualize the rivet 500. The elongate body portion 510 includes the first expansion zone 504 and the second expansion zone 506, with a tissue capture zone 510a therebetween. Each of the expansion zones 504, 506 is pre-formed or otherwise biased to "roll" into an open configuration (similar to, for example, the rolling action used in rolling down socks on a person's leg) using a memory material such as, for example, nitinol. During an application of the rivet 500, the rivet 500 is held in its unexpanded configuration by an outer sleeve (not shown) that may be withdrawn when the rivet 500 is in a desired position, in order to allow expansion of the first and second expansion zones 504, 506.

FIG. 5B depicts a single-expanded configuration of the rivet 500, wherein the first expansion zone 504 has been expanded to form a first securement region 512. FIG. 5C depicts a double-expanded configuration of the rivet 500, wherein in addition to the first expansion zone 504 having been expanded, the second expansion zone 506 has also been expanded to form a second securement region 514. In this embodiment, the first and second expansion zones 504, 506 are biased into an open configuration, but they may alternatively be configured to be rolled outward by a longitudinal compression of the elongate body portion 510 of the rivet 500 to expand to the position shown in FIG. 5C. FIG. 5D shows a longitudinal cross-section of FIG. 5C taken along a line 5D-5D, in diagrammatic fashion. As shown therein, the first and second expansion zones 504, 506 are "curled open" or "rolled open" on either side of the tissue capture zone 510a.

Those of skill in the art will appreciate that, in a method of applying a rivet 500, a central mandrel is not needed to expand the rivet. A central mandrel may be used to retain the rivet in a delivery sheath until desired deployment/expansion, and an outer sheath may be used to restrain the rivet 500 from deploying/expanding until it is in a desired position. Thus, deployment of a rivet 500 may be effected in a manner similar to that shown in FIGS. 3A-3F, except that the mandrel 250 plays a longitudinal retaining role, and expansion is accomplished by releasing the first or second expansion zone 504, 506 from the outer sheath 230 - either by retracting the outer sheath 230, or by using the inner sheath 240 to advance a portion of the rivet 500 distally past the distal end of the outer sheath 230 where the rivet 500 is able to open into its biased-open shape to form the first or second securement region 512, 514.

Those of skill in the art will appreciate that the different petal/ expansion zone configurations shown and described may be combined with each other or with other petal/ expansion zone embodiments within the scope of the present invention. Those of skill in the art will appreciate that other embodiments of the rivet introduction device and system described herein may also be practiced within the scope of the present invention. It is therefore intended that the foregoing detailed description be regarded as illustrative rather than limiting. It should be understood that the following claims are intended to define the scope of this invention.

## Claims

1. A medical device configured for introducing a blind rivet through soft tissue, the device comprising:
a rivet introduction device (202) comprising:
an elongate outer sheath (230) having an outer sheath lumen (232) extending therethrough;
an elongate inner sheath (240) having an inner sheath lumen (242) extending therethrough; and
an elongate mandrel (250) having a guide wire lumen (252) extending therethrough;
wherein the elongate inner sheath is disposed in a longitudinally slidable manner through at least a portion of the outer sheath lumen and the elongate mandrel is disposed in a longitudinally slidable manner through at least a portion of the inner sheath lumen;
wherein a distal end portion of the elongate mandrel includes flexible structure configured to allow radial expansion/contraction; the device further comprising:
a rivet (100) disposed coaxially around the elongate mandrel adjacent the distal end portion of the elongate mandrel,
the rivet comprising a rivet lumen having an inner diameter, and wherein an outer diameter of the rivet is greater than an inner diameter of the inner sheath lumen; and a guide wire adapted to be extended through the guide wire lumen of the mandrel;
**characterised in that** the distal end portion of the elongate mandrel comprises a plurality of separated jaw members (254) arranged such that when the guide wire extends through the distal end portion of the elongate mandrel an outer diameter of the distal end portion of the elongate mandrel is expanded from being smaller to be larger than the inner diameter of the rivet lumen.

2. The medical device of claim 1, wherein the rivet comprises first and second expansion zones.

3. The medical device of claim 2, wherein the rivet comprises a generally cylindrical shape with a rivet wall surrounding the rivet lumen, the rivet lumen open at a proximal end and a distal end of the rivet.

4. The medical device of claim 3, wherein one of the first and second expansion zones of the rivet comprises a series of generally parallel apertures through the rivet wall nearer the distal end of the rivet, and the other of the first and second expansion zones comprises a series of generally parallel apertures through the rivet wall nearer the proximal end of the rivet.

5. The medical device of claim 4, wherein at least one of the generally parallel apertures of the rivet extends to the proximal end of the rivet.

6. The medical device of claim 4, wherein at least one of the generally parallel apertures of the rivet extends to the distal end of the rivet.

7. The medical device of claim 3, wherein each of the first and second expansion zones is configured to expand to an outer diameter that is greater than an outer diameter of the rivet wall upon application of a longitudinal force to the rivet.

8. The medical device of claim 2, wherein at least one of the first and second expansion zones of the rivet is biased into an expanded configuration.

9. The medical device of claim 8, wherein the at least one of the first and second expansion zones of the rivet assumes the expanded configuration when not circumferentially restrained by the elongate outer sheath.

## Patentansprüche

1. Medizinische Vorrichtung zur Einführung einer Blindniete durch Weichgewebe, wobei die Vorrichtung Folgendes umfasst:
eine Nieteneinführungsvorrichtung (202), die Folgendes umfasst:
eine längliche Außenhülle (230) mit einem sich durch sie hindurch erstreckenden Außenhüllenlumen (232);
eine längliche Innenhülle (240) mit einem sich durch sie hindurch erstreckenden Innenhüllenlumen (242); und
einen länglichen Mandrin (250) mit einem sich durch ihn hindurch erstreckenden Führungsdrahtlumen (252);
worin die längliche Innenhülle auf eine in Längsrichtung gleitende Weise durch zumindest einen Teil des Außenhüllenlumens angeordnet ist und der längliche Mandrin auf eine in Längsrichtung gleitende Weise durch zumindest einen Teil des Innenhüllenlumens angeordnet ist;
worin ein distaler Endabschnitt des länglichen Mandrins eine flexible Konstruktion aufweist, die radiale Expansion/Kontraktion zulässt; wobei die Vorrichtung ferner eine Niete (100) umfasst, die koaxial um den länglichen Mandrin neben dem distalen Endabschnitt des länglichen Mandrins angeordnet ist, wobei die Niete ein Nietenlumen mit einem Innendurchmesser aufweist und
worin ein Außendurchmesser der Niete größer ist als ein Innendurchmesser des Innenhüllenlumens; und einen Führungsdraht, der durch das Führungsdrahtlumen des Mandrins ausgezogen werden kann;
**dadurch gekennzeichnet, dass** der distale Endabschnitt des länglichen Mandrins eine Vielzahl von getrennten Backenelementen (254) umfasst, die so angeordnet sind, dass, wenn sich der Führungsdraht durch den distalen Endabschnitt des länglichen Mandrins erstreckt, ein Außendurchmesser des distalen Endabschnitts des länglichen Mandrins so erweitert wird, dass er nicht mehr kleiner sondern größer ist als der Innendurchmesser des Nietenlumens.

2. Medizinische Vorrichtung nach Anspruch 1, worin die Niete erste und zweite Expansionszonen umfasst.

3. Medizinische Vorrichtung nach Anspruch 2, worin die Niete eine allgemein zylinderförmige Gestalt umfasst und eine Nietenwand das Nietenlumen umgibt, wobei das Nietenlumen an einem proximalen Ende und einem distalen Ende der Niete offen ist.

4. Medizinische Vorrichtung nach Anspruch 3, worin die erste und/oder zweite Expansionszone der Niete eine Reihe von allgemein parallelen Öffnungen durch die Nietenwand näher am distalen Ende der Niete umfasst und die jeweils andere der ersten und zweiten Expansionszonen eine Reihe von allgemein parallelen Öffnungen durch die Nietenwand näher am proximalen Ende der Niete umfasst.

5. Medizinische Vorrichtung nach Anspruch 4, worin sich zumindest eine der allgemein parallelen Öffnungen der Niete bis zum proximalen Ende der Niete erstreckt.

6. Medizinische Vorrichtung nach Anspruch 4, worin sich zumindest eine der allgemein parallelen Öffnungen der Niete bis zum distalen Ende der Niete erstreckt.

7. Medizinische Vorrichtung nach Anspruch 3, worin die ersten und zweiten Expansionszonen jeweils so konfiguriert sind, dass sie sich bei Applikation einer Längskraft auf die Niete auf einen Außendurchmesser aufweiten können, der größer ist als ein Außendurchmesser der Nietenwand.

8. Medizinische Vorrichtung nach Anspruch 2, worin zumindest die erste und/oder die zweite Expansionszone der Niete in eine aufgeweitete Konfiguration vorgespannt ist.

9. Medizinische Vorrichtung nach Anspruch 8, worin zumindest die erste und/oder die zweite Expansionszone der Niete die aufgeweitete Konfiguration annimmt, wenn sie nicht am Umfang von der länglichen Außenhülle gehalten wird.

## Revendications

1. Dispositif médical configuré pour introduire un rivet aveugle dans du tissu mou, le dispositif comportant :
un dispositif (202) d'introduction de rivets comportant :
une gaine (230) extérieure allongée présentant une lumière (232) de gaine extérieure la traversant ;
une gaine (240) intérieure allongée présentant une lumière (242) de gaine intérieure la traversant ; et
un mandrin (250) allongé présentant une lumière (252) pour fil de guidage le traversant ;
dans lequel la gaine intérieure allongée est disposée de manière à coulisser longitudinalement dans au moins une section de la lumière de la gaine extérieure et le mandrin allongé est disposé de manière à coulisser longitudinalement dans au moins une section de la lumière de la gaine intérieure ;
dans lequel une section d'extrémité distale du mandrin allongé comprend une structure souple et configurée pour permettre une expansion/contraction radiale ; le dispositif comportant en outre :
un rivet (100) disposé de manière coaxiale autour du mandrin allongé en position adjacente à la section d'extrémité distale du mandrin allongé, le rivet comportant une lumière de rivet possédant un diamètre intérieur, et dans lequel un diamètre extérieur du rivet est supérieur à un diamètre intérieur de la lumière de la gaine intérieure ; et un fil de guidage conçu pour s'étendre dans la lumière pour fil de guidage du mandrin ;
**caractérisé en ce que** la section d'extrémité distale du mandrin allongé comporte une pluralité d'éléments (254) mâchoires séparés disposés de telle sorte que lorsque le fil de guidage s'étend dans la section d'extrémité distale du mandrin allongé, un diamètre extérieur de la section d'extrémité distale du mandrin allongé est élargi d'une taille plus petite à une taille plus grande que le diamètre intérieur de la lumière de rivet.

2. Dispositif médical selon la revendication 1, dans lequel le rivet comporte une première et une seconde zones d'expansion.

3. Dispositif médical selon la revendication 2, dans lequel le rivet comporte une forme globalement cylindrique avec une paroi de rivet entourant la lumière de rivet, la lumière de rivet étant ouverte au niveau d'une extrémité proximale et d'une extrémité distale du rivet.

4. Dispositif médical selon la revendication 3, dans lequel une desdites première et seconde zones d'expansion du rivet comporte une série d'ouvertures globalement parallèles à travers la paroi de rivet située le plus près de l'extrémité distale du rivet, et l'autre desdites première et seconde zones d'expansion comporte une série d'ouvertures globalement parallèles à travers la paroi de rivet située le plus près de l'extrémité proximale du rivet.

5. Dispositif médical selon la revendication 4, dans lequel au moins une desdites ouvertures globalement parallèles du rivet s'étend vers l'extrémité proximale du rivet.

6. Dispositif médical selon la revendication 4, dans lequel au moins une desdites ouvertures globalement parallèles du rivet s'étend vers l'extrémité distale du rivet.

7. Dispositif médical selon la revendication 3, dans lequel chacune desdites première et seconde zones d'expansion est configurée pour s'élargir jusqu'à un diamètre extérieur qui est supérieur à un diamètre extérieur de la paroi de rivet au moment de l'application d'une force longitudinale sur le rivet.

8. Dispositif médical selon la revendication 2, dans lequel au moins une desdites première et seconde zones d'expansion du rivet est sollicitée dans une configuration élargie.

9. Dispositif médical selon la revendication 8, dans lequel ladite au moins une desdites première et seconde zones d'expansion du rivet prend la configuration élargie lorsqu'elle n'est pas retenue circonférentiellement par la gaine extérieure allongée.
